# FASCICULE DE BREVET EUROPEEN

(11) **EP 2 675 488 B1**
(45) Date de publication et mention de la délivrance du brevet: **07.04.2021**
(21) Numéro de dépôt: 12709926.5
(22) Date de dépôt: 17.02.2012
(51) Int. Cl.: A61L 9/12, A61L 9/03, A01M 1/00, F24F 13/00, A01M 13/00, F24F 3/16

(54) **PROCEDE DE FABRICATION D'UN MELANGE GAZEUX POUR LE TRAITEMENT D'ATMOSPHERES VICIEES, DISPOSITIF POUR SA MISE EN OEUVRE ET UTILISATION**
VERFAHREN ZUR HERSTELLUNG EINER GASMISCHUNG ZUR BEHANDLUNG VON VERUNREINIGTEN ATMOSPHÄREN, VORRICHTUNG ZUR UMSETZUNG DES VERFAHRENS UND SEINE VERWENDUNG
METHOD FOR MANUFACTURING A GASEOUS MIXTURE FOR TREATING POLLUTED ATMOSPHERES, DEVICE FOR IMPLEMENTING SAME, AND USE THEREOF

(30) Priorité: 17.02.2011 FR 1151317
(43) Date de publication de la demande: 25.12.2013
(73) Titulaire: Air&D (Société À Responsabilité Limitée), 67560 Rosheim (FR)
(72) Inventeur: WUEST, Robert, F-67560 Rosheim (FR); VASKO, Miroslav, F-67560 Rosheim (FR)
(74) Mandataire: Cabinet Nuss
(86) Numéro de dépôt international: PCT/FR2012/050343
(87) Numéro de publication internationale: WO 2012/110750

(56) Documents cités:
- EP-A1- 2 071 947
- WO-A1-02/25180
- WO-A1-2004/030708
- WO-A1-2005/092400
- US-A1- 2008 233 001

## Description

La présente invention concerne le domaine du traitement d'atmosphères malodorantes, en particulier d'atmosphères chargées en substances nauséabondes et/ou nuisibles à la santé humaine ou animale, et concerne plus particulièrement la désodorisation, le parfumage, l'assainissement (par exemple la désinfection) et/ou la désinsectisation de l'air tel qu'il peut être présent sur des sites industriels ou privés. A cet effet, elle a pour objet un procédé pour enrichir, de préférence saturer, de l'air atmosphérique ordinaire en substances actives présentes en phase gazeuse, en particulier en substances volatiles de désodorisation, de parfumage, de désinfection et/ou de désinsectisation, un dispositif pour la mise en œuvre dudit procédé et l'utilisation dudit procédé pour traiter de l'air vicié. En effet, un tel mélange gazeux enrichi ou saturé permettra ensuite de traiter efficacement une atmosphère viciée en la désodorisant, la parfumant en la rendant saine et/ou inoffensive (exempte de polluants, d'agents pathogènes ou d'insectes vecteurs de maladies ou de nuisances) pour les personnes qui la respirent.

On connaît des procédés et des dispositifs destinés à créer un mélange gazeux à base d'air atmosphérique ordinaire contenant des substances ou principes actifs de désodorisation, de parfumage et/ou d'assainissement d'air ambiant (par exemple US 2008/0233001 A1, WO 02/25180 A1 etc.).

La demande EP 1 593 397 A1 décrit un dispositif en forme de colonne comprenant un gel sur lequel sont adsorbés des substances actives diffusant dans un flux d'air ascendant créé par un ventilateur situé à la base de ladite colonne.

La demande EP 2 071 947 A1 propose un dispositif et procédé de ces types, dans lesquels de l'air ordinaire sain est aspiré depuis l'environnement puis mis en contact avec des substances actives à pression atmosphérique constante, la quantité d'air prélevée correspondant, à tout moment, à la quantité d'air chargée en substances actives quittant ledit dispositif pour être acheminée vers les sites où il est utilisé pour traiter l'air vicie.

Ces dispositifs ne fournissent toutefois pas des résultats optimaux dans la mesure où lesdites substances actives volatiles ne sont entraînées que très partiellement sous l'effet du passage unique du flux d'air frais créé à cet effet. L'air sortant de ces dispositifs n'est donc que relativement peu chargée en substances actives et donc que peu efficace et ne sont donc peu ou pas adaptés au traitement de grands volumes ou de volumes d'air très pollués.

La présente invention vise à pallier les inconvénients précités.

A cet effet elle a pour objet un procédé pour enrichir, de préférence saturer, de l'air ordinaire en substances actives présentes en phase gazeuse, en particulier en substances volatiles de désodorisation, de parfumage, de désinfection et/ou de désinsectisation, caractérisé en ce qu'il consiste à réaliser, dans une enceinte étanche, les opérations suivantes :
a) introduire de l'air frais gazeux non chargé en substances actives dans ladite enceinte,
b) l'introduire par au moins une entrée dans au moins une chambre d'enrichissement ou de saturation, pour mettre en contact ledit air frais avec au moins une substance active volatile présente en phase gazeuse afin de l'enrichir, de préférence jusqu'à saturation, en ladite ou lesdites substances actives volatiles,
c) prélever, à la sortie de ladite ou desdites chambres d'enrichissement ou de saturation, une portion de mélange gazeux enrichi à un taux suffisant ou saturé à l'issue de l'étape b) en vue de son stockage dans une chambre de stockage interne ou de son évacuation vers l'extérieur via un ou plusieurs orifices de sortie de ladite enceinte,
d) réinjecter la portion de mélange gazeux non-prélevée à l'étape c) ou l'intégralité dudit mélange gazeux enrichi dans ladite au moins une chambre d'enrichissement ou de saturation afin d'augmenter le taux d'enrichissement dudit mélange gazeux, de le maintenir à son niveau maximal de saturation ou de l'abaisser en le mélangeant, dans ce cas, avant l'introduction dans ladite au moins une chambre d'enrichissement ou de saturation avec du nouvel air frais non chargé introduit depuis l'extérieur dans ladite enceinte.

Selon une caractéristique avantageuse, le procédé selon la présente invention est caractérisé en ce que l'air frais gazeux non chargé en substances actives est introduit dans ladite enceinte par aspiration, de préférence en étant filtré, au moyen d'au moins un premier dispositif d'aspiration, de préférence au moyen d'un premier ventilateur au niveau d'au moins un orifice d'entrée de ladite enceinte.

Avantageusement, la température de l'air frais non chargé entrant et/ou la température du mélange gazeux est réglée au moyen d'un dispositif de chauffage et de régulation, et de préférence maintenue à environ 25 °C.

Selon une autre caractéristique, la mise en contact gazeux, dans au moins une chambre d'enrichissement ou de saturation, dudit air frais gazeux avec ladite au moins une substance active volatile en phase gazeuse afin d'enrichir ledit air frais, de préférence jusqu'à saturation, en ladite ou lesdites substances actives volatiles en phase gazeuse, se fait en faisant circuler ledit air frais ou le mélange gazeux réinjecté de l'étape d) sur, sous et/ou le long d'une ou plusieurs surfaces chargées et/ou à travers un ou plusieurs corps volumiques chargés en une ou plusieurs substances actives volatiles de désodorisation, de parfumage, de désinfection et/ou de désinsectisation et qui diffusent ainsi, sous forme gazeuse, pour enrichir ou saturer ledit air frais ou le mélange gazeux réinjecté.

De façon préférée, la ou lesdites surfaces et/ou le ou lesdits corps volumiques sont chauffés directement ou indirectement de façon réglable au moyen d'un dispositif de chauffage réglable afin de réguler la quantité de substance active volatile diffusée ou prélevée par unité de temps et de surface.

Avantageusement, le prélèvement à l'au moins une sortie de ladite ou desdites chambres d'enrichissement ou de saturation, d'une portion de mélange gazeux enrichi s'effectue par aspiration au moyen d'un ou de plusieurs dispositifs d'aspiration, de préférence au moyen d'un ou de plusieurs compresseurs.

De manière avantageuse, le prélèvement de l'étape c) s'effectue lorsque l'enrichissement du mélange gazeux en la substance active en phase gazeuse en question est supérieur ou égal à 50%, de préférence supérieur ou égal à 70% et plus préférentiellement supérieur ou égal à 95% du taux maximal théorique de saturation de l'air en ladite substance active.

Selon une autre caractéristique, le rapport entre le flux d'air frais gazeux non chargé entrant et le ou les flux de mélange gazeux enrichi ou saturé sortant de l'enceinte est supérieur à 1,5, de préférence compris entre 1,5 et 4, ou plus préférentiellement compris entre 4 et 8.

De manière particulièrement pratique, la réinjection de l'étape d) du mélange gazeux enrichi ou saturé avec du nouvel air frais gazeux non chargé est réalisée au moyen d'au moins un second dispositif d'aspiration, de préférence au moyen d'un second ventilateur.

Un autre aspect intéressant réside dans le fait que le procédé selon l'invention est caractérisé en ce que l'on régule le flux gazeux généré par le au moins premier et/ou second dispositif d'aspiration, de préférence le premier et/ou second ventilateur, ce afin de contrôler la quantité de substance active volatile diffusée ou prélevée par unité de temps et de surface.

Selon une autre variante, l'introduction d'air frais gazeux non chargé s'effectue en continue pendant tout le cycle d'enrichissement ou de saturation.

Avantageusement, la quantité et/ou le moment d'introduction d'air frais gazeux non chargé et/ou de prélèvement de mélange gazeux enrichi ou saturé sont programmables.

La présente invention a également pour objet un dispositif pour la mise en œuvre du procédé selon l'invention, caractérisé en ce qu'il comprend une enceinte étanche, munie d'au moins un orifice d'entrée et d'au moins un orifice de sortie communiquant tous deux avec l'extérieur de ladite enceinte, d'un compartiment principal alimenté, au niveau d'une première zone de ce dernier par ledit au moins un orifice en air frais extérieur non chargé en substances actives au moyen d'au moins un premier dispositif d'aspiration, de préférence d'au moins un premier ventilateur et muni, en son sein, d'au moins un second dispositif d'aspiration, de préférence d'au moins un second ventilateur aspirant les gaz contenus dans ledit compartiment principal vers au moins une entrée d'au moins une chambre d'enrichissement ou de saturation desdits gaz en substances actives volatiles, la ou les sorties de la ou des chambres d'enrichissement ou de saturation débouchant dans une seconde zone dudit compartiment principal qui est géographiquement éloignée de ladite première zone et du ou des orifices d'entrée et qui est munie, en son sein, d'au moins un compresseur pour prélever au moins une portion de mélange gazeux enrichi ou saturé formé, à destination d'une chambre de stockage interne, d'un consommateur ou d'un réservoir de stockage intermédiaire externes via l'au moins un orifice de sortie, ladite seconde zone étant en communication gazeuse avec ladite première zone de sorte à permettre une réinjection du mélange gazeux enrichi ou saturé formé dans la au moins une chambre d'enrichissement ou de saturation, le cas échéant avec l'air frais non chargé entrant par le ou les orifices d'entrée.

Selon une autre caractéristique, le dispositif selon l'invention est caractérisé en ce qu'il comporte au moins un moyen de chauffage et de régulation de la température de l'air frais extérieur non chargé en substances actives et/ou des gaz déjà présents dans le compartiment principal.

Le dispositif selon l'invention est encore caractérisé en ce que la ou les chambres d'enrichissement ou de saturation comportent au moins une surface chargée et/ou au moins un corps volumique chargé en substance(s) active(s) volatiles de désodorisation, de parfumage, de désinfection et/ou de désinsectisation, de sorte à pouvoir diffuser, sous forme gazeuse, pour enrichir ou saturer ledit air frais ou le mélange gazeux réinjecté.

Selon une mode de réalisation préféré, la ou les chambres d'enrichissement ou de saturation sont réalisées sous la forme de contenants étanches de forme allongée à l'intérieur desquels sont disposés la ou les surfaces chargées et/ou le ou les corps volumiques chargés en substance(s) active(s) répartis sur toute l'étendue longitudinale desdits contenants, de préférence sur au moins deux niveaux parallèles plans et décalés l'un de l'autre dans le sens de la hauteur.

Selon une autre caractéristique, la ou les chambres d'enrichissement ou de saturation présentent des chicanes.

Avantageusement, le dispositif selon l'invention est caractérisé en ce que la ou les chambres d'enrichissement ou de saturation présentent des moyens de chauffage réglables de la ou des surfaces chargées et/ou du ou des corps chargés en substance(s) active(s).

Dans une autre variante avantageuse, le dispositif conforme à la présente invention est caractérisé en ce qu'il présente plusieurs seconds ventilateurs, chacun étant relié à une chambre d'enrichissement ou de saturation qui lui est propre, chaque chambre d'enrichissement ou de saturation présentant sa propre sortie débouchant dans ou reliée à ladite seconde zone munie d'au moins un compresseur pour prélever au moins une portion de mélange gazeux enrichi ou saturé, et un organe de commande et de gestion desdits plusieurs seconds ventilateurs et dudit ou desdits compresseurs.

Enfin, l'invention a également pour objet l'utilisation du procédé selon l'invention caractérisée en ce que le mélange gazeux enrichi ou saturé obtenu par la mise en œuvre dudit procédé est mélangé, selon une quantité efficace, avec l'air vicié à traiter pour le désodoriser, le parfumer, l'assainir et/ou de débarrasser des insectes qu'il contient.

L'invention sera mieux comprise, grâce à la description ci-après, qui se rapporte à des modes de réalisation préférés, donnés à titre d'exemples non limitatifs, et expliqués avec référence aux dessins schématiques annexés, dans lesquels :
- la figure 1 représente, de façon schématique et simplifiée, une coupe en élévation latérale d'un premier mode de réalisation d'un dispositif selon l'invention,
- la figure 2 représente, de façon schématique et simplifiée, une coupe en élévation latérale d'un second mode de réalisation d'un dispositif selon l'invention, et
- la figure 3 représente, de façon schématique et simplifiée, une coupe en élévation latérale d'un troisième mode de réalisation d'un dispositif selon l'invention.

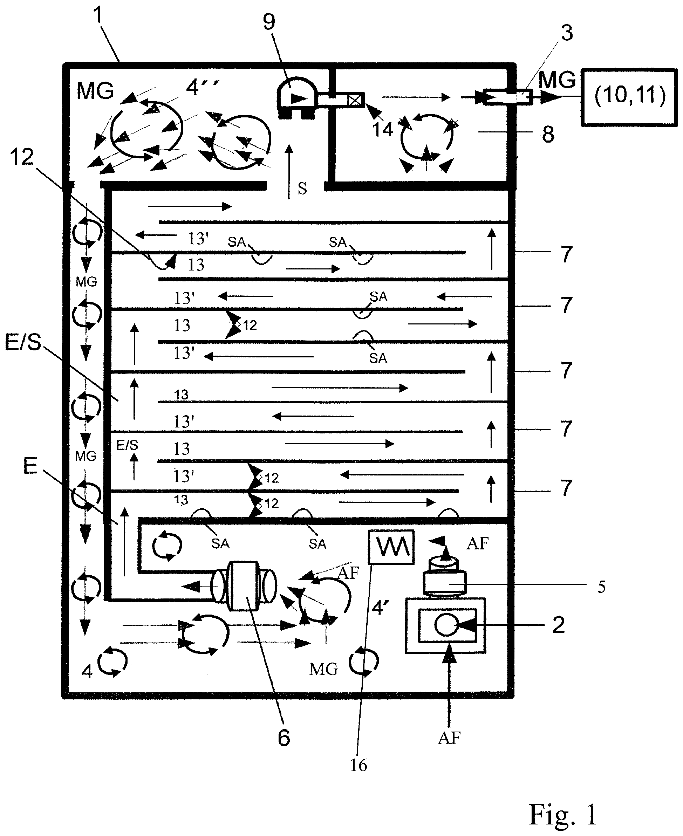

En se référant aux figures 1 à 3 susvisés, la présente invention a donc pour objet un procédé pour enrichir, de préférence saturer, de l'air ordinaire en substances actives SA présentes en phase gazeuse, en particulier en substances volatiles de désodorisation, de parfumage, de désinfection et/ou de désinsectisation, caractérisé en ce qu'il consiste à réaliser, dans une enceinte 1 étanche, les opérations suivantes :
a) introduire de l'air frais AF gazeux non chargé en substances actives dans ladite enceinte 1,
b) l'introduire par au moins une entrée E dans au moins une chambre d'enrichissement ou de saturation 7, pour mettre en contact ledit air frais AF avec au moins une substance active SA volatile présente en phase gazeuse afin de l'enrichir, de préférence jusqu'à saturation, en ladite ou lesdites substances actives SA volatiles,
c) prélever, à la sortie S de ladite ou desdites chambres d'enrichissement ou de saturation 7, une portion de mélange gazeux MG enrichi à un taux suffisant ou saturé à l'issue de l'étape b) en vue de son stockage dans une chambre de stockage interne 8 ou de son évacuation vers l'extérieur via un ou plusieurs orifices de sortie 3 de ladite enceinte 1,
d) réinjecter la portion de mélange gazeux MG non-prélevée à l'étape c) ou l'intégralité dudit mélange gazeux MG enrichi ou saturé dans ladite au moins une chambre d'enrichissement ou de saturation 7 afin d'augmenter le taux d'enrichissement dudit mélange gazeux MG, de le maintenir à son niveau maximal de saturation ou de l'abaisser en le mélangeant, dans ce cas, avant l'introduction dans ladite au moins une chambre d'enrichissement ou de saturation 7 avec du nouvel air frais AF non chargé introduit depuis l'extérieur dans ladite enceinte 1.

Le procédé selon l'invention permet donc de fabriquer, à partir d'air ordinaire, soit de l''air atmosphérique normal, c'est-à-dire à partir un air naturel suffisamment propre et sain donc suffisamment dépourvu de polluants atmosphériques odorants ou non (industriels ou naturels), de germes pathogènes ou d'insectes vecteurs de tels germes, un air de traitement particulièrement chargé ou riche en substances actives destinées à un traitement de désodorisation, de parfumage, de désinfection et/ou de désinsectisation en vue de traiter un air malodorant et/ou vicié. Cet air suffisamment pur pour être utilisable dans le cadre de la présente invention peut être prélevé in situ ou provenir de réservoirs de stockage adaptés, par exemple d'air pressurisé.

Par opposition on qualifiera de "vicié" tout air impur ou pollué qui nécessite un traitement de purification, désodorisation, et/ou décontamination (assainissement).

Par "air frais" on entend, dans le cadre de la présente invention, de l'air extérieur au procédé ou dispositif selon l'invention qui n'est pas chargé en substances actives et dont la qualité ou le degré de pureté soit suffisant pour pouvoir être utilisé.

Grâce au procédé selon l'invention, l'air chargé obtenu peut être tellement enrichi, voire saturé en substances actives, que l'efficacité du procédé de purification de l'air vicié proprement s'en trouve considérablement améliorée, tant sur le plan technique (durées de traitement réduites, résultats qualitativement meilleurs) que sur le plan économique (coûts d'installation, de traitement et d'entretien réduits).

Comme substances actives SA qui devront être présentes en phase gazeuse, on utilisera en particulier les substances volatiles de désodorisation, de parfumage, de désinfection et/ou de désinsectisation habituellement utilisées et connues de l'homme du métier, ces dernières comprenant aussi bien les substances odorantes masquant (notamment par superposition ou saturation) simplement les mauvaises odeurs que les substances chimiquement et/ou physiquement réactives qui agissent sur les molécules malodorantes et/ou organismes indésirables pour les neutraliser voire les détruire, par exemple par modification, décomposition ou inactivation physique ou chimique.

On citera à titre d'exemples non limitatifs de substances actives notamment les familles chimiques des aldéhydes, des terpènes, des esters, des insecticides, biocides, désodorants et d'autres composés connus tels que ceux cités, par exemple, dans la demande EP-A-1 334 735.

La mise en contact, à l'intérieur de la ou des chambres d'enrichissement ou de saturation 7, de l'air frais AF gazeux avec les substances actives SA volatiles permet d'enrichir ledit air frais AF en lesdites substances actives jusqu'à obtenir un mélange gazeux MG suffisamment concentré, voire saturé en lesdites substances SA. Cette mise en contact ou échange en phase gazeuse se fait en faisant passer l'air frais AF gazeux sur une surface 12 d'échange bidimensionnelle (par exemple une plaque surfacique enduite d'une couche de substance active SA) ou tridimensionnelle d'un support volumique (par exemple un corps volumique céramique poreux) chargés en substances actives SA de laquelle ou duquel ces dernières peuvent être libérées, par exemple par diffusion, évaporation, entraînement ou analogue.

Ce phénomène de diffusion peut notamment être favorisé ou contrôlé par le choix de substances actives SA particulièrement volatiles, en augmentant la température des gaz participant à l'échange, en augmentant la surface active d'échange, en utilisant de l'air frais AF peu ou pas chargé, en augmentant la pression (le flux) avec lequel l'air frais AF non chargé ou le mélange gazeux MG déjà partiellement chargé en substances passe sur les surfaces ou supports volumiques chargés et entre en contact avec lesdites substances actives SA ou en combinant plusieurs des mesures précités.

Comme modules chargés en substances actives SA adaptés à la présente invention on peut citer, par exemple, des cassettes en forme de plaques commercialisés sous la dénomination « AIR FORCE » par la société Biothys GmbH à Willstädt en Allemagne. Ces cassettes prêtes à l'emploi consistent en des plaques de substances actives capturées (de façon relargable) dans un gel.

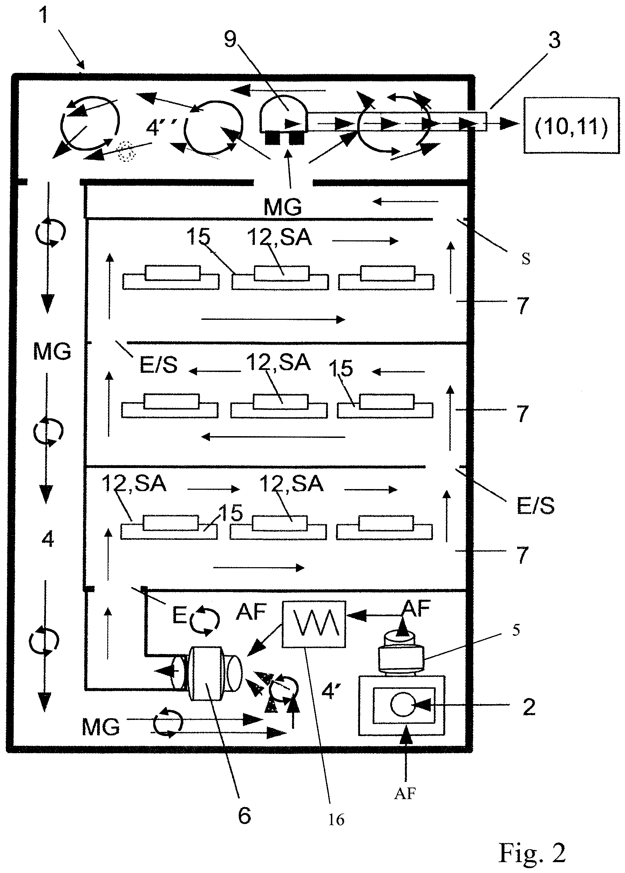

A la sortie S de la ou des chambres d'enrichissement ou de saturation 7, c'est-à-dire à la fin du circuit d'enrichissement ou de saturation qu'auront traversés l'air frais AF non chargé (lorsqu'il entre pour la première fois dans ledit circuit) ou le mélange gazeux MG enrichi ou (lorsque de l'air déjà enrichi précédemment a été réinjecté dans le circuit), on prélève, si cela est possible et souhaité, une portion dudit mélange gazeux MG suffisamment enrichi (ou saturé) afin de le stocker, de préférence sous pression, dans le but de créer une réserve d'air de traitement prêt à l'emploi dans une chambre de stockage 8 interne ou un réservoir de stockage 11 externe ou afin de l'envoyer vers un consommateur 10 où il peut être directement utilisé, par exemple pour désodoriser un site. Ladite ou lesdites chambres de stockage 8 internes seront avantageusement réalisées de manière occuper un volume total pouvant typiquement aller de 100 1 à 1000 1, et seront conçues pour résister à une pression de plusieurs bar (typiquement jusqu'à 15 bar). De façon préférée, chaque h et munie d'une soupape ou valve anti-retour 14 comme suggérée à titre d'exemple sur la figure 2 pour éviter tout reflux dans la seconde zone 4" de l'enceinte 1.

Avantageusement, on évitera de prélever l'intégralité du mélange gazeux MG formé, au moins temporairement, afin de permettre la recirculation des gaz dans le circuit d'enrichissement ou de saturation qui se met en place à l'intérieur de l'enceinte 1.

En effet, la portion de mélange gazeux MG non-prélevée à l'étape c) ou l'intégralité dudit mélange gazeux MG enrichi ou saturé est réinjectée dans ladite au moins une chambre d'enrichissement ou de saturation 7 afin d'augmenter encore le taux d'enrichissement dudit mélange gazeux MG, de le maintenir à son niveau maximal de saturation déjà atteint ou de l'abaisser en le mélangeant, dans ce cas, avant son introduction dans ladite au moins une chambre d'enrichissement ou de saturation 7 avec du nouvel air frais AF non chargé introduit depuis l'extérieur dans ladite enceinte 1.

Selon une caractéristique avantageuse, le procédé selon l'invention est caractérisé en ce que l'air frais AF gazeux non chargé en substances actives SA est introduit dans ladite enceinte 1 par aspiration, de préférence en étant filtré, au moyen d'au moins un premier dispositif d'aspiration, de préférence au moyen d'un premier ventilateur 5 au niveau d'au moins un orifice d'entrée 2 de ladite enceinte 1.

D'autres moyens bien connus de l'homme du métier peuvent également être utilisés comme moyens d'aspiration, par exemple des pompes. Toutefois, l'utilisation de ventilateurs s'avère plus commode et plus économique et est donc préférée. L'utilisation de filtres adaptés permet d'éviter l'aspiration d'objets indésirables tels que des corps étrangers, sable, poussières, pollens, graminées, insectes et analogues qui risquent l'entraver le bon fonctionnement de l'installation. Des filtres physiques et/ou chimiques supplémentaires peuvent également être employés si l'air dont on dispose n'est pas suffisamment pur ou propre.

De manière particulièrement avantageuse, la température de l'air frais AF non chargé entrant et/ou la température du mélange gazeux MG à l'intérieur de l'enceinte 1 est réglée au moyen d'un dispositif de chauffage et de régulation, et de préférence maintenue à environ 25 °C.

Ainsi on garantit un fonctionnement, en particulier un échange gazeux entre les gaz et les substances actives volatilisées qui soit aussi constant et performant que possible.

Une régulation de la température est particulièrement utile et recommandée lorsque le procédé est mis en œuvre dans un environnement climatique variable, par exemple dans des régions où la température varie beaucoup au cours du temps de la mise en œuvre du procédé, par exemple au cours d'une journée ou au gré des saisons.

Comme évoqué ci-dessus, le procédé selon l'invention est encore caractérisé en ce que la mise en contact gazeux, dans au moins une chambre d'enrichissement ou de saturation 7, dudit air frais AF gazeux avec ladite au moins une substance active SA volatile en phase gazeuse afin d'enrichir ledit air frais AF, de préférence jusqu'à saturation, en ladite ou lesdites substances actives SA volatiles en phase gazeuse, se fait en faisant circuler ledit air frais AF ou le mélange gazeux MG réinjecté de l'étape d) sur, sous et/ou le long d'une ou plusieurs surfaces 12 chargées et/ou à travers au moins un corps volumique chargé en une ou plusieurs substances actives SA volatiles de désodorisation, de parfumage, de désinfection et/ou de désinsectisation et qui diffusent ainsi, sous forme gazeuse, pour enrichir ou saturer ledit air frais AF ou le mélange gazeux MG réinjecté.

La qualité de l'échange gazeux entre l'air frais AF ou le mélange gazeux MG déjà enrichi avec la ou les substances actives SA conditionne l'efficacité de la production d'air de traitement et doit donc être la meilleure possible. Comme déjà indiqué, l'augmentation de la surface d'échange efficace permet d'optimiser cet échange. On veillera donc à garantir un contact entre les différents gaz aussi important que possible en s'assurant que les gaz à enrichir passent bien sur les surfaces 12 pour se charger au mieux en matière actives SA ou qu'ils traversent bien ceux-ci, dans le cas de corps volumiques, en s'imprégnant bien et emportant dans leur flux lesdites substances.

Avantageusement, on peut prévoir que la ou lesdites surfaces 12 et/ou les corps volumiques traversés sont chauffées directement ou indirectement de façon réglable au moyen d'un dispositif de chauffage réglable afin de réguler la quantité de substance active SA volatile diffusée ou prélevée par unité de temps et de surface.

On pourra utiliser tous les dispositifs et mécanismes de réglages habituels connus de l'homme du métier, qui permettent d'ajuster automatiquement une valeur de température mesurée à une consigne prédéfinie en vue d'obtenir une certaine libération de substance(s) active(s) SA par unité de temps et de surface ou exprimée en masse de substance(s) active(s) libérée(s) de son support surfacique (plaques de gel chargées) ou volumique (corps poreux chargé) par heure. L'homme du métier saura notamment déterminer sans difficulté la quantité substance(s) active(s) SA à libérer pour obtenir une teneur donnée pour un flux gazeux défini.

Selon une autre caractéristique, le prélèvement, à l'au moins une sortie S de ladite ou desdites chambres d'enrichissement ou de saturation 7, d'une portion de mélange gazeux MG enrichi s'effectue par aspiration au moyen d'un ou de plusieurs dispositifs d'aspiration, de préférence au moyen d'un ou de plusieurs compresseurs 9.

D'autres moyens bien connus de l'homme du métier peuvent également être utilisés comme moyens d'aspiration, par exemple des pompes. Toutefois, l'utilisation de compresseurs s'avère plus performante puisque l'air de traitement prêt à l'emploi est généralement comprimé en vue de son stockage ou de son acheminement vers son lieu d'utilisation et est donc préférée.

De façon préférée, le prélèvement de l'étape c) s'effectue lorsque l'enrichissement du mélange gazeux MG en la substance active SA en phase gazeuse en question est supérieur ou égal à 50%, de préférence supérieur ou égal à 70% et plus préférentiellement supérieur ou égal à 95% du taux maximal théorique de saturation de l'air en ladite substance active SA.

On obtient ainsi des gaz de traitement à différentes teneurs en substances actives SA mais dont la concentration est avantageusement relativement voire beaucoup plus importante que celle des gaz obtenus avec des procédés ou dispositifs selon l'état de la technique, ces derniers présentant habituellement des teneurs en substances actives que de l'ordre de quelques % du taux maximal théorique de saturation de l'air en ladite substance active SA.

Selon une autre caractéristique, le procédé selon l'invention est remarquable en ce que le rapport entre le flux d'air frais AF gazeux non chargé entrant et le ou les flux de mélange gazeux MG enrichi ou saturé sortant de l'enceinte 1 est supérieur à 1,5, de préférence compris entre 1,5 et 4, ou plus préférentiellement compris entre 4 et 8.

Cette différence de flux créée en ajustant les paramètres opérationnels (puissance, débit...) des moyens d'aspiration d'air au niveau de la ou des entrées 2 et de la ou des sorties 3 de l'enceinte 1 permet de maintenir les gaz circulant dans le circuit d'enrichissement ou de saturation à l'intérieur de l'enceinte 1 sous pression de sorte à favoriser l'extraction des substances actives SA dans les chambres 7 et l'homogénéité des mélanges gazeux obtenus, en particulier au niveau de l'entrée d'air gazeux frais AF non chargé.

Comme pour l'aspiration au niveau de l'entrée 2, la réinjection de l'étape d) du mélange gazeux MG enrichi ou saturé avec du nouvel air frais AF gazeux non chargé est avantageusement réalisée au moyen d'au moins un second dispositif d'aspiration, de préférence au moyen d'un second ventilateur 6.

Afin de pouvoir piloter le procédé et de l'adapter aux besoins de l'utilisateur, on prévoit de réguler le flux gazeux généré par le au moins premier et/ou second dispositif d'aspiration, de préférence le premier et/ou second ventilateur 5, 6, ce afin de contrôler la quantité de substance active SA volatile diffusée ou prélevée par unité de temps et de surface.

Selon un mode de réalisation particulier, l'introduction d'air frais AF gazeux non chargé s'effectue en continue pendant tout le cycle d'enrichissement ou de saturation.

On peut également prévoir un mode ou l'entrée d'air frais AF gazeux non chargé est interrompue pendant une partie du cycle d'enrichissement ou de saturation, notamment lorsqu'une teneur spécifique souhaitée est atteinte, par exemple une teneur maximale de saturation, et que la capacité de l'installation a produit suffisamment de mélange gazeux MG saturé pour répondre aux besoins pendant la période d'interruption donnée. L'alimentation en air frais AF pourra alors reprendre dès que les stocks ne suffisent plus et qu'il faille relancer la production de mélange gazeux de traitement saturé.

Avantageusement la quantité et/ou le moment d'introduction d'air frais AF gazeux non chargé et/ou de prélèvement de mélange gazeux MG enrichi ou saturé sont programmables, par exemple au moyen de tout système de temporisation et d'actionnement classique connu de l'homme du métier. On peut ainsi répondre à des demandes variant dans le temps ou préprogrammer des phases de productions / d'arrêt lorsqu'une quantité particulièrement importante de mélange gazeux doit être produite ou que le procédé doit être arrêté, par exemple pour des raisons de maintenance.

L'invention a également pour objet un dispositif pour la mise en œuvre du procédé. En relation avec les figures 1 ou 2 qui montrent deux exemples de dispositifs selon l'invention, on peut voir qu'un tel dispositif est caractérisé en ce qu'il comprend une enceinte 1 étanche, munie d'au moins un orifice d'entrée 2 et d'au moins un orifice de sortie 3 communiquant tous deux avec l'extérieur de ladite enceinte 1, d'un compartiment principal 4 alimenté, au niveau d'une première zone 4' de ce dernier par ledit au moins un orifice 2 en air frais AF extérieur non chargé en substances actives SA au moyen d'au moins un premier dispositif d'aspiration, de préférence d'au moins un premier ventilateur 5 et muni, en son sein, d'au moins un second dispositif d'aspiration, de préférence d'au moins un second ventilateur 6 aspirant les gaz contenus dans ledit compartiment principal 4 vers au moins une entrée E d'au moins une chambre d'enrichissement ou de saturation 7 desdits gaz en substances actives SA volatiles, la ou les sorties S de la ou des chambres d'enrichissement ou de saturation 7 débouchant dans une seconde zone 4" dudit compartiment principal 4 qui est géographiquement éloignée de ladite première zone 4' et du ou des orifices d'entrée 2 et qui est munie, en son sein, d'au moins un compresseur 9 pour prélever au moins une portion de mélange gazeux MG enrichi ou saturé formé, à destination d'une chambre de stockage interne 8, d'un consommateur 10 ou d'un réservoir de stockage 11 intermédiaire externes via l'au moins un orifice de sortie 3, ladite seconde zone 4" étant en communication gazeuse avec ladite première zone 4' de sorte à permettre une réinjection du mélange gazeux MG enrichi ou saturé formé dans la au moins une chambre d'enrichissement ou de saturation 7, le cas échéant avec l'air frais AF non chargé entrant par le ou les orifices d'entrée 2.

On entend par "géographiquement éloignée" le fait que la seconde zone 4" soit suffisamment distante de la première zone 4' et des orifices d'entrée 2 pour ne pas perturber le bon fonctionnement du dispositif.

Dans l'exemple représenté aux figures précitées, on a représenté une enceinte 1 munie d'une seule entrée et d'une seule sortie. Une telle enceinte 1 peut être une armoire étanche en acier, par exemple en acier inox ou en aluminium, dont les dimensions sont naturellement adaptées à la quantité de mélange gazeux MG devant être produite. A titre d'exemple concret une enceinte 1 de petite taille mesurera environ 2 mètres de haut pour une largeur et une profondeur également d'environ 2 m, soit un volume brut de 8 m³.

L'orifice d'entrée 2 et de sortie 3 sont munis de joints ad hoc (non représentés) afin d'éviter ou de minimiser au maximum toute fuite de mélange gazeux MG vers l'extérieur.

L'air frais AF extérieur (exempt de toute substance active SA) et de qualité suffisante est aspiré à travers l'orifice d'entrée 2 par le moyen d'aspiration réalisé ici sous la forme d'un premier ventilateur 5 pouvant être muni d'un filtre (non représenté), par exemple, d'un filtre à insectes, d'un filtre à sable, à particules.

L'orifice d'entrée 2 est de préférence situé dans la partie basse de l'enceinte 1 proche du sol sur lequel elle repose.

Selon une autre caractéristique, le dispositif selon l'invention comporte au moins un moyen de chauffage et de régulation de la température de l'air frais AF extérieur non chargé en substances actives SA et/ou des gaz déjà présents dans le compartiment principal 4. Comme représenté, l'air frais AF gazeux qui pénètre ainsi dans l'enceinte 1, au niveau de la première zone 4' passe sur ou à proximité d'un radiateur 16 réglable, par exemple de 500 W sous 230 V / 50 Hz, qui le réchauffe le cas échéant jusqu'à la température souhaitée, par exemple de 25 °C. D'autres radiateurs réglables (non représentés) peuvent être prévus comme moyens de chauffage et de régulation des gaz circulant à l'intérieur de l'enceinte 1 pour optimiser les mélanges et la libération des substances actives SA déposées sur les surfaces 12 ou emprisonnées dans les corps volumiques chargées.

Pour des raisons de clarté, et sauf indication contraire, les connexions électriques notamment d'alimentation, les capteurs ou sondes de températures, les moyens de régulation, l'électronique de commande et de gestion du dispositif, les vannes et joints d'étanchéité etc. n'ont pas été ou pas tous été représentés sur les figures qui restent des schémas simplifiés de principe, l'homme du métier sachant de lui-même exécuter toute mesure constructive habituelle qui s'avère nécessaire.

L'air frais AF ainsi porté à la bonne température est aspiré, toujours au niveau de la partie basse de l'enceinte 1, par un second dispositif d'aspiration réalisé ici sous la forme d'un second ventilateur 6. A titre indicatif, le premier et second ventilateur ont chacun un débit de fonctionnement normal de l'ordre de 170 m³/h pour une puissance de l'ordre de 40 W sous 230 V / 50 Hz.

Dans la première zone 4', à proximité de l'entrée dudit ventilateur 6, de l'air frais non chargé se mélange normalement, après un certain temps de fonctionnement du dispositif, avec du mélange gazeux MG déjà formé (c'est-à-dire de l'air frais AF qui a déjà traversé une chambre d'enrichissement ou de saturation et qui s'est déjà chargé en substances actives SA) et donc et provenant, sous l'effet de l'aspiration, de la seconde zone 4" du compartiment 4 située dans la partie haute de l'enceinte 1 à la sortie S de la ou des chambres d'enrichissement ou de saturation 7.

Le mélange gazeux MG ainsi formé alimente, via une canalisation dédiée, une première chambre d'enrichissement ou de saturation 7 autonome dans laquelle il pénètre par une entrée E, qu'il traverse selon une direction horizontale de gauche à droite sur la figure 1 sur une distance correspondant grossièrement à la largeur du dispositif ou de l'enceinte 1 pour remonter un coude et retraverser, de la droite vers la gauche un second compartiment similaire au premier qu'il vient de traverser pour quitter ainsi ladite chambre au niveau d'une sortie S à laquelle peut immédiatement être raccordée une deuxième, nouvelle chambre d'enrichissement ou de saturation 7, laquelle peut elle aussi, à son tour être prolongée d'une troisième nouvelle chambre d'enrichissement ou de saturation 7 du même type, etc.

Dans l'exemple représenté à la figure 1 on peut ainsi dénombrer cinq chambres d'enrichissement ou de saturation 7 se suivant en série, la dernière sortie S se situant alors dans la partie haute de l'enceinte 1 au niveau de la seconde zone 4" où le volume de mélange gazeux MG produit peut, lorsqu'il est suffisamment enrichi ou saturé, être ponctionné par le compresseur 9 et stocké dans la chambre de stockage interne 8 ou quitter l'enceinte via l'orifice de sortie 3.

Dans l'exemple représenté à la figure 2, ce sont trois chambres d'enrichissement ou de saturation 7 qui se suivent en série, la dernière sortie S se situant également dans la partie haute de l'enceinte 1 au niveau de la seconde zone 4". Dans cet exemple, lesdites chambres 7 ne présentent qu'un compartiment que le flux de mélange gazeux MG ne traverse qu'une fois avant de pénétrer dans la chambre suivante. On notera que la direction du flux gazeux de la gauche vers la droite pour la première chambre s'inverse lors de la traversée de la chambre suivante et que le chemin parcouru par ledit mélange gazeux MG le long d'une chambre d'enrichissement ou de saturation 7 correspond en gros à la largeur de l'enceinte 1.

Ces exemples illustratifs montrent comment tirer partie du volume global de l'enceinte 1 pour augmenter et optimiser la longueur du chemin parcouru par le mélange gazeux et donc le temps de contact des différents gaz avec les surfaces 12 et/ou corps volumiques chargés en substances actives qu'elles balayent ou traversent pour les enrichir jusqu'à les saturer en la ou les substances actives. En effet, lors de leur passage à travers les chambres d'enrichissement ou de saturation 7, l'air frais AF non encore chargé et/ou le mélange gazeux déjà préalablement enrichi par un premier passage entre en contact avec les substances actives SA volatiles et se charge plus ou moins de celles-ci, en fonction notamment, de la nature des substances actives SA, du gaz à enrichir, du temps de contact, de la température, du degré de saturation du gaz présent.

Le degré d'enrichissement ou de saturation du mélange gazeux MG sortant de la (dernière) chambre d'enrichissement ou de saturation 7 peut être mesuré par prélèvement, par l'intermédiaire d'une sonde adaptée placée dans l'enceinte ou évaluée empiriquement. Si la teneur en substances actives SA n'est pas suffisante, le mélange gazeux MG ne sera pas prélevé par le compresseur 9 et poursuivra automatiquement son chemin dans le compartiment 4 vers la première zone 4', aspiré par le second ventilateur 6 comme représenté schématiquement sur la partie de gauche des figures 1 ou 2. Là, il se mélangera le cas échéant avec du nouvel air frais AF ou sera à nouveau aspiré dans la conduite menant à la première chambre d'enrichissement ou de saturation 7 pour un nouveau passage. Le cycle reprend ainsi jusqu'à l'obtention d'un mélange gazeux MG dont le taux d'enrichissement est satisfaisant.

A titre d'information, le compresseur 9 possède, en mode normal de fonctionnement, un débit de l'ordre de seulement 16 m³/h pour une puissance de l'ordre de 210 W sous 230 V / 50 Hz alors que le premier et second ventilateur 5, 6 ont chacun un débit de fonctionnement normal de l'ordre de 170 m³/h.

Comme évoqué plus haut, le dispositif selon la présente invention est caractérisé en ce que la ou les chambres d'enrichissement ou de saturation 7 comportent au moins une surface 12 chargée et/ou au moins un corps volumique chargé en substance(s) active(s) SA volatiles de désodorisation, de parfumage, de désinfection et/ou de désinsectisation, de sorte à pouvoir diffuser, sous forme gazeuse, pour enrichir ou saturer ledit air frais AF ou le mélange gazeux MG réinjecté.

Selon le mode de réalisation représenté aux figures 1 et 2, la ou les chambres d'enrichissement ou de saturation 7 sont réalisées sous la forme de contenants étanches de forme allongée à l'intérieur desquels sont disposés la ou les surfaces 12 chargées en substance(s) active(s) SA réparties sur toute l'étendue longitudinale desdits contenants, de préférence sur au moins deux niveaux 13, 13' parallèles plans et décalés l'un de l'autre dans le sens de la hauteur (figure 1).

Par "forme allongée" on entend une forme de contenant dont la section ou la largeur présente une dimension inférieure à celle de la longueur dudit contenant.

La ou les substances actives SA peuvent notamment être déposées comme schématiquement suggéré sur la ou les surfaces 12, par exemple sous la forme de monticules de matière isolés, de plaques de gel enduites déposées, de couches de substances actives appliquées ou analogue. Les surfaces 12 peuvent être les parois des chambres d'enrichissement ou de saturation 7 proprement dites ou rapportées sur ces dernières. La ou les surfaces 12 chargées, par exemple des plaques fines de gel imprégné, et/ou les corps volumiques chargés en substances actives SA, par exemple des corps poreux en céramique ou zéolithes, peuvent également être déposés sur des supports 15, réalisés par exemple sous la forme de tiroirs ou tablettes coulissables, montés à l'intérieur de chaque chambre d'enrichissement ou de saturation 7 comme le montre la figure 2, de sorte à laisser un espace suffisamment grand entre lesdites surfaces 12 ou corps volumique et les parois de ladite chambre afin que les flux de gaz puissent les balayer efficacement et ainsi permettre la libération et l'entraînement desdites substances actives SA. Avantageusement, les supports 15 sont réalisés de façon à permettre un accès optimal des gaz aux substances actives SA, en particulier en y prévoyant suffisamment d'orifices. Lesdits supports 15 peuvent ainsi être réalisés sous la forme de grilles, de filets ou de treillis assurant un bon contact gaz-substances actives SA. L'échange, le remplacement, ou le réapprovisionnement en substances actives SA desdits supports 15 est également facilité lorsque ces derniers sont réalisés sous la forme de tiroirs coulissables faciles d'accès et pouvant être aisément manipulés.

Afin que la circulation de l'air ou du mélange gazeux soit suffisante, un espacement d'au moins 5 mm peut par exemple être prévu entre deux plaques de gel parallèles du type « AIR FORCE » mentionnées plus haut.

Avantageusement, la ou les chambres d'enrichissement ou de saturation 7 présentent des chicanes. Ceci permet également d'augmenter le temps de séjour des gaz dans lesdites chambres d'enrichissement ou de saturation 7.

Selon une variante non représentée, la ou les chambres d'enrichissement ou de saturation 7 présentent des moyens de chauffage réglables des surfaces 12 chargées et/ou des corps volumiques chargés en substance(s) active(s) SA.

Ce chauffage permet d'améliorer et/ou de contrôler la libération ou diffusion des substances actives SA liquides ou solides dans le mélange de gaz devant être enrichi ou saturé. Il peut s'effectuer de manière directe, par exemple en chauffant directement les substances actives SA, par exemple en chauffant leur support ou récipient qui les contient ou indirectement en chauffant l'environnement direct desdites substances actives SA qui transmettra alors sa chaleur aux supports et/ou aux substances actives précités.

Ce chauffage peut notamment être réalisé par un radiateur placé à proximité des substances actives SA à chauffer, en particulier par un radiateur destiné à chauffer l'air ou le mélange gazeux MG à enrichir. On peut aussi prévoir de chauffer directement les plaques de gel chargé, par exemple en chauffant les supports ou surfaces métalliques sur lesquels elles reposent ou dans lesquels elles sont enchâssées.

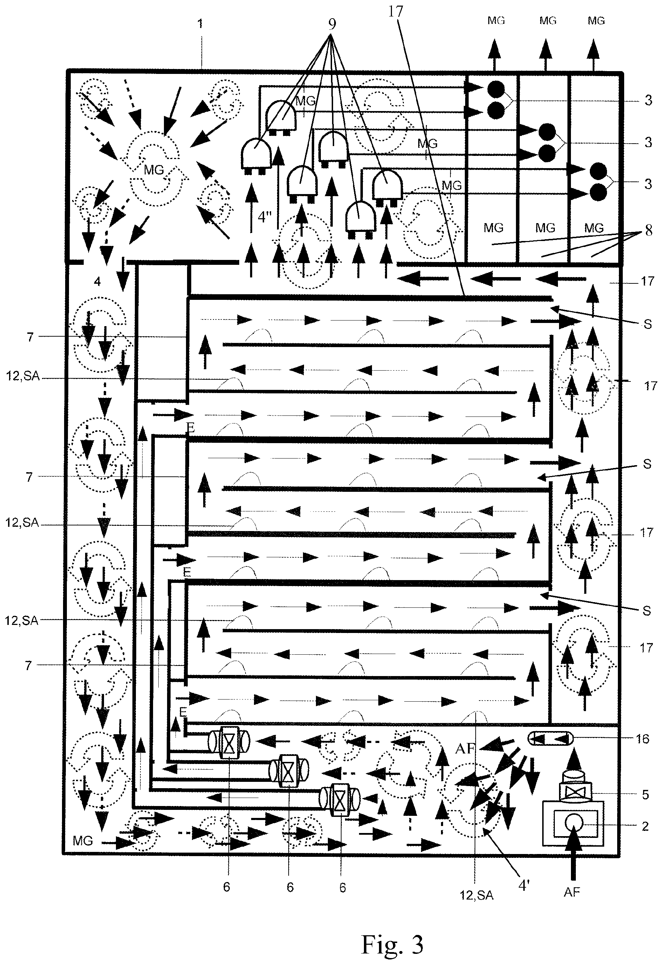

Selon un troisième exemple de réalisation représenté à la figure 3, le dispositif selon la présente invention est caractérisé en ce qu'il présente plusieurs (trois) seconds ventilateurs 6, chacun étant relié à une chambre d'enrichissement ou de saturation 7 qui lui est propre par une canalisation, chaque chambre d'enrichissement ou de saturation 7 présentant sa propre sortie S débouchant dans ou reliée, par l'intermédiaire ici d'une conduite collectrice 17, à ladite seconde zone 4" munie d'au moins un compresseur 9 pour prélever au moins une portion de mélange gazeux MG enrichi ou saturé, et un organe de commande et de gestion desdits plusieurs seconds ventilateurs 6 et dudit ou desdits compresseurs 9. Ledit mélange prélevé est évacué de l'enceinte 1 par les six compresseurs 9 via les six orifices de sortie 3.

Il faut veiller à ce que les débits ou flux générés par lesdits seconds ventilateurs restent supérieurs à ceux cumulés des compresseurs 9 de manière à ce qu'il règne toujours une surpression à l'intérieur de l'enceinte 1. Cette surpression permet une meilleure libération des substances actives SA et un bon mélange et une bonne recirculation des gaz à l'intérieur de ladite enceinte 1. Dans l'exemple de la figure 3, la somme maximale des flux prélevés par les six compresseurs 9 est de 9 x 16 = 96 m³/h pour 170 m³/h de flux venant de l'extérieur (air frais AF) ou de flux de mélange gazeux MG et éventuellement d'air frais AF aspiré par chaque ventilateur 6 de capacité identique et égale à celle du premier ventilateur 5.

Bien entendu, toutes les combinaisons possibles sont envisageables par l'homme du métier en ce qui concerne la réalisation du dispositif selon l'invention au niveau des chambres d'enrichissement ou de saturation 7 qui peuvent être disposées en série l'une après l'autre et/ou en parallèle l'une à côté de l'autre. L'homme du métier saura prévoir des moyens de connexion et d'alimentation et d'extraction adaptés des gaz circulants de sorte à créer le circuit de recirculation propre à la présente invention qui permet au cours des passages successifs des gaz à les enrichir ou les saturer en substances actives SA avant qu'ils soient prélevés pour être stockés ou utilisés dans le traitement d'atmosphères malodorantes et/ou infestées.

Le nombre effectif de recirculations du gaz à l'intérieur de l'enceinte 1, c'est-à-dire le nombre de passages du mélange gazeux MG dans la ou les chambres d'enrichissement ou de saturation 7 dépend évidemment de nombreux paramètres et en particulier du degré de saturation en substances actives SA que l'on souhaite obtenir. A titre d'exemple, le mélange gazeux MG peut devoir effectuer 30 passages avant d'atteindre une teneur de 100% du maximal théorique atteignable en quantité de substances actives SA chargées. Une consommation en substances actives SA de 3 g/h est indiquée comme point de repère.

La présente invention a encore pour objet, l'utilisation du procédé selon l'invention caractérisée en ce que le mélange gazeux MG enrichi ou saturé obtenu par la mise en œuvre dudit procédé est mélangé, selon une quantité efficace, avec l'air vicié à traiter pour le désodoriser, le parfumer, l'assainir et/ou de débarrasser des insectes (mouches, moustiques...) qu'il contient.

Par "quantité efficace" on entend, une quantité suffisante pour désodoriser, parfumer, assainir et/ou décontaminer l'air vicié, soit complètement, soit selon le degré souhaité et/ou en fonction de l'application envisagée.

L'homme du métier saura adapter, par exemple selon la méthode dite des essais et erreurs, les quantités d'air à traiter et de mélange gazeux MG, notamment en fonction du degré de pollution dudit air et de la concentration en substances actives du mélange gazeux MG.

A titre d'exemples non limitatifs d'atmosphères ou d'environnements pouvant être traités on peut citer :
- les cheminées industrielles,
- les installations de fabrication de gaz naturel,
- les décharges, déchetteries, ou halles de compostage,
- les égouts et réseaux de canalisation,
- les stations d'épuration, en particulier les bassins de décantation,
- les sites industriels produisant des mauvaises odeurs ou des odeurs fortes et incommodantes (industrie papetière, chimique, usines d'équarrissage, sites d'élevage industriel, conserveries de poissons...),
- les atmosphères publiques plus ou moins confinées à parfumer (magasins, salles de réunions, théâtres, halles de gares ou d'aéroports, trains, avions, couloirs de métro...) ou à désodoriser (restaurants, stations-services, vestiaires sportifs...), et
- les sites naturels particulièrement odorants (marécages, eaux stagnantes...).

La technologie selon l'invention est également particulièrement utile pour traiter des zones dites « ATEX » (atmosphères explosives) en installant un dispositif selon l'invention en dehors de cette zone et en prévoyant des gaines ou canalisations adaptées permettant d'alimenter lesdites zones en mélange gazeux MG pour le traitement de leurs atmosphères. Le dispositif selon l'invention peut être installé à proximité immédiate desdites zones ou à de grandes distances de celles-ci (300 m à plusieurs kilomètres), ce qui en fait un outil particulièrement flexible et pratique. Cela permet également d'opérer une centralisation des dispositifs de fabrication du mélange gazeux MG traitant qui peut ensuite être distribué à l'aide d'un réseau de canalisations ad hoc. Une telle distribution est également particulièrement avantageuse lorsqu'il s'agit de traiter, non pas une zone de faible étendue localisée de façon compacte (par exemple le volume d'air interne bien défini d'un hangar d'élevage industriel) mais l'ensemble d'un réseau de volumes s'étendant de façon linéaire et/ou ramifiée, tel que notamment un réseau d'égouts ou de couloirs souterrains. Dans ce dernier cas, les gaines ou conduites véhiculant le mélange gazeux MG peuvent être munis, par exemple à intervalles réguliers, de moyens de diffusion (ouvertures, buses, ventilateurs...), de préférence réglables et/ou programmables, dudit mélange gazeux MG dans l'environnement à traiter.

## Revendications

1. Procédé pour enrichir, de préférence saturer, de l'air atmosphérique ordinaire, appelé ci-après air frais (AF), en substances actives (SA) présentes en phase gazeuse, en substances volatiles de désodorisation, de parfumage, de désinfection et/ou de désinsectisation, **caractérisé en ce qu'**il consiste à réaliser, dans une enceinte (1) étanche, les opérations suivantes :
a) introduire de l'air frais (AF) gazeux non chargé en substances actives dans ladite enceinte (1), au moyen d'un premier ventilateur (5) au niveau d'au moins un orifice d'entrée (2) de ladite enceinte (1),
b) l'introduire par au moins une entrée (E) dans plusieurs chambres d'enrichissement ou de saturation (7), pour mettre en contact ledit air frais (AF) avec au moins une substance active (SA) volatile présente en phase gazeuse afin de l'enrichir, de préférence jusqu'à saturation, en ladite ou lesdites substances actives (SA) volatiles,
c) prélever, à la sortie (S) desdites chambres d'enrichissement ou de saturation (7), une portion de mélange gazeux (MG) enrichi à un taux suffisant ou saturé à l'issue de l'étape b), au moyen d'un ou de plusieurs compresseur(s) (9), en vue de son stockage dans une chambre de stockage interne (8) ou de son évacuation vers l'extérieur via un ou plusieurs orifices de sortie (3) de ladite enceinte (1),
d) réinjecter la portion de mélange gazeux (MG) non-prélevée à l'étape c) ou l'intégralité dudit mélange gazeux (MG) enrichi ou saturé dans lesdits chambres d'enrichissement ou de saturation (7) au moyen d'un second ventilateur (6) afin d'augmenter le taux d'enrichissement dudit mélange gazeux (MG), de le maintenir à son niveau maximal de saturation ou de l'abaisser en le mélangeant, dans ce cas, avant l'introduction dans lesdites chambres d'enrichissement ou de saturation (7) avec du nouvel air frais (AF) non chargé introduit depuis l'extérieur dans ladite enceinte (1), le rapport entre le flux d'air frais (AF) gazeux non chargé entrant et le ou les flux de mélange gazeux (MG) enrichi ou saturé sortant de l'enceinte (1) étant supérieur à 1,5 et,
e) réguler le flux gazeux généré par le premier et/ou second ventilateur (5, 6), ce afin de contrôler la quantité de substance active (SA) volatile diffusée ou prélevée par unité de temps et de surface.

2. Procédé selon la revendication 1, **caractérisé en ce que** l'air frais (AF) gazeux non chargé en substances actives est introduit dans ladite enceinte (1) en étant filtré.

3. Procédé selon la revendication 1 ou 2, **caractérisé en ce que** la température de l'air frais (AF) non chargé entrant et/ou la température du mélange gazeux (MG) est réglée au moyen d'un dispositif de chauffage et de régulation, et de préférence maintenue à environ 25 °C.

4. Procédé selon l'une quelconque des revendications 1 à 3, **caractérisé en ce que** la mise en contact gazeux, dans les chambres d'enrichissement ou de saturation (7), dudit air frais (AF) gazeux avec ladite au moins une substance active (SA) volatile en phase gazeuse afin d'enrichir ledit air frais (AF), de préférence jusqu'à saturation, en ladite ou lesdites substances actives (SA) volatiles en phase gazeuse, se fait en faisant circuler ledit air frais (AF) ou le mélange gazeux (MG) réinjecté de l'étape d) sur, sous et/ou le long d'une ou plusieurs surfaces (12) et/ou à travers un ou plusieurs corps volumiques chargés en une ou plusieurs substances actives (SA) volatiles de désodorisation, de parfumage, de désinfection et/ou de désinsectisation et qui diffusent ainsi, sous forme gazeuse, pour enrichir ou saturer ledit air frais (AF) ou le mélange gazeux (MG) réinjecté.

5. Procédé selon la revendication 4, **caractérisé en ce que** la ou lesdites surfaces (12) et/ou le ou les corps volumiques sont chauffés directement ou indirectement de façon réglable au moyen d'un dispositif de chauffage réglable afin de réguler la quantité de substance active (SA) volatile diffusée ou prélevée par unité de temps et de surface.

6. Procédé selon l'une quelconque des revendications 1 à 5, **caractérisé en ce que** le prélèvement de l'étape c) s'effectue lorsque l'enrichissement du mélange gazeux (MG) en la substance active (SA) en phase gazeuse en question est supérieur ou égal à 50%, de préférence supérieur ou égal à 70% et plus préférentiellement supérieur ou égal à 95% du taux maximal théorique de saturation de l'air en ladite substance active (SA).

7. Procédé selon l'une quelconque des revendications 1 à 6, **caractérisé en ce que** le rapport entre le flux d'air frais (AF) gazeux non chargé entrant et le ou les flux de mélange gazeux (MG) enrichi ou saturé sortant de l'enceinte (1) est compris entre 1,5 et 4, ou préférentiellement compris entre 4 et 8.

8. Procédé selon l'une quelconque des revendications 1 à 7, **caractérisé en ce que** l'introduction d'air frais (AF) gazeux non chargé s'effectue en continue pendant tout le cycle d'enrichissement ou de saturation.

9. Dispositif pour la mise en œuvre du procédé selon l'une quelconque des revendications précédentes, ledit dispositif comprenant une enceinte (1) étanche, munie d'au moins un orifice d'entrée (2) et d'au moins un orifice de sortie (3) communiquant tous deux avec l'extérieur de ladite enceinte (1), d'un compartiment principal (4) alimenté, au niveau d'une première zone (4') de ce dernier par ledit au moins un orifice (2) en air frais (AF) extérieur non chargé en substances actives (SA) au moyen d'au moins un premier ventilateur (5) et muni, en son sein, d'au moins un second ventilateur (6) aspirant les gaz contenus dans ledit compartiment principal (4) vers au moins une entrée (E) de plusieurs chambres d'enrichissement ou de saturation (7) desdits gaz en substances actives (SA) volatiles, et un moyen de régulation du flux gazeux généré par ledit au moins premier et/ou ledit au moins second ventilateur (5, 6), **caractérisé en ce que** la ou les sorties (S) desdites chambres d'enrichissement ou de saturation (7) débouchant dans ou reliée à une seconde zone (4") dudit compartiment principal (4) qui est géographiquement éloignée de ladite première zone (4') et du ou des orifices d'entrée (2) et qui est munie, en son sein, d'au moins un compresseur (9) pour prélever au moins une portion de mélange gazeux (MG) enrichi ou saturé formé, à destination d'une chambre de stockage interne (8), d'un consommateur (10) ou d'un réservoir de stockage (11) intermédiaire externes via l'au moins un orifice de sortie (3), ladite seconde zone (4") étant en communication gazeuse avec ladite première zone (4') de sorte à permettre une réinjection du mélange gazeux (MG) enrichi ou saturé formé dans lesdites chambres d'enrichissement ou de saturation (7), le cas échéant avec l'air frais (AF) non chargé entrant par le ou les orifices d'entrée (2).

10. Dispositif selon la revendication 9, **caractérisé en ce qu'**il comporte au moins un moyen de chauffage et de régulation de la température de l'air frais (AF) extérieur non chargé en substances actives (SA) et/ou des gaz déjà présents dans le compartiment principal (4).

11. Dispositif selon la revendication 9 ou 10, **caractérisé en ce qu'**une ou plusieurs desdites chambres d'enrichissement ou de saturation (7) comportent au moins une surface (12) chargée et/ou au moins un corps volumique chargé en substance(s) active(s) (SA) volatiles de désodorisation, de parfumage, de désinfection et/ou de désinsectisation, de sorte à pouvoir diffuser, sous forme gazeuse, pour enrichir ou saturer ledit air frais (AF) ou le mélange gazeux (MG) réinjecté.

12. Dispositif selon la revendication 11, **caractérisé en ce qu'**une ou plusieurs desdites chambres d'enrichissement ou de saturation (7) sont réalisées sous la forme de contenants étanches de forme allongée à l'intérieur desquels sont disposés la ou les surfaces (12) et/ou le ou les corps volumiques chargés en substance(s) active(s) (SA) répartis sur toute l'étendue longitudinale desdits contenants, de préférence sur au moins deux niveaux (13, 13') parallèles plans et décalés l'un de l'autre dans le sens de la hauteur.

13. Dispositif selon la revendication 11 ou 12, **caractérisé en ce qu'**une ou plusieurs desdites chambres d'enrichissement ou de saturation (7) présentent des chicanes.

14. Dispositif selon l'une quelconque des revendications 11 à 13, **caractérisé en ce qu'**une ou plusieurs desdites chambres d'enrichissement ou de saturation (7) présentent des moyens de chauffage réglables des surfaces (12) ou corps volumiques chargés en substance(s) active(s) (SA).

15. Dispositif selon l'une quelconque des revendications 9 à 14, **caractérisé en ce qu'**il présente plusieurs seconds ventilateurs (6), chacun étant relié à une chambre d'enrichissement ou de saturation (7) qui lui est propre, chaque chambre d'enrichissement ou de saturation (7) présentant sa propre sortie (S) débouchant dans ou reliée à ladite seconde zone (4") munie d'au moins un compresseur (9) pour prélever au moins une portion de mélange gazeux (MG) enrichi ou saturé, et un organe de commande et de gestion desdits plusieurs seconds ventilateurs (6) et dudit ou desdits compresseurs (9).

16. Utilisation du procédé selon l'une quelconque des revendications 1 à 8, **caractérisée en ce que** le mélange gazeux (MG) enrichi ou saturé obtenu par la mise en œuvre dudit procédé est mélangé, selon une quantité efficace, avec l'air vicié à traiter pour le désodoriser, le parfumer, l'assainir et/ou de débarrasser des insectes qu'il contient.

## Patentansprüche

1. Verfahren zum Anreichern, vorzugsweise Sättigen, von gewöhnlicher Umgebungsluft, die nachstehend als Frischluft (AF) bezeichnet wird, mit in der Gasphase vorliegenden aktiven Stoffen (SA), mit flüchtigen Stoffen zur Beseitigung unangenehmer Gerüche, zur Beduftung, zur Desinfektion und/oder Insektenbekämpfung, **dadurch gekennzeichnet, dass** es darin besteht, in einem dichten Raum (1) folgende Vorgänge durchzuführen:
a) Einleiten von gasförmiger Frischluft (AF), in der keine aktiven Stoffe enthalten sind, in den Raum (1) mit einem ersten Lüfter (5) im Bereich von mindestens einer Einströmöffnung (2) des Raums (1),
b) Einleiten derselben über mindestens einen Einlass (E) in mehrere Anreicherungs- oder Sättigungskammern (7), damit die Frischluft (AF) mit mindestens einem in der Gasphase vorliegenden flüchtigen aktiven Stoff (SA) zusammengebracht wird, damit sie mit dem oder den flüchtigen aktiven Stoff(en) (SA), vorzugsweise bis zur Sättigung, angereichert wird,
c) am Auslass (S) der Anreicherungs- oder Sättigungskammern (7) Entnehmen eines Teils des Gasgemischs (MG), das am Ende von Schritt b) in einer ausreichenden Menge angereichert oder gesättigt ist, mit einem oder mehreren Verdichter(n) (9) zwecks Speicherung desselben in einer inneren Speicherkammer (8) oder Ableitung desselben über eine oder mehrere Ausströmöffnungen (3) des Raums (1) nach außen,
d) erneutes Einleiten des nicht in Schritt c) entnommenen Teils des angereicherten oder gesättigten Gasgemischs (MG) oder des gesamten angereicherten oder gesättigten Gasgemischs (MG) in die Anreicherungs- oder Sättigungskammern (7) mit einem zweiten Lüfter (6) zur Erhöhung der Anreicherung des Gasgemischs (MG), zum Halten derselben auf dem höchsten Sättigungsniveau oder zum Absenken derselben, wobei es in diesem Fall vor dem Einleiten in die Anreicherungs- oder Sättigungskammern (7) mit neuer, keine aktiven Stoffe enthaltenden Frischluft (AF) gemischt wird, die von außen in den Raum (1) geleitet wird, wobei das Verhältnis zwischen dem einströmenden Strom aus gasförmiger Frischluft (AF), die keine aktiven Stoffe enthält, und dem Strom oder den Strömen aus angereichertem oder gesättigtem Gasgemisch (MG), das aus dem Raum (1) strömt, über 1,5 beträgt, und
e) Regeln des Gasstroms, der von dem ersten und/oder zweiten Lüfter (5, 6) erzeugt wird, damit die pro Zeiteinheit und Flächeneinheit diffundierte oder entnommene Menge an flüchtigem aktivem Stoff (SA) gesteuert wird.

2. Verfahren nach Anspruch 1, **dadurch gekennzeichnet, dass** die gasförmige Frischluft (AF), die keine aktiven Stoffe enthält, in den Raum (1) geleitet und dabei gefiltert wird.

3. Verfahren nach Anspruch 1 oder 2, **dadurch gekennzeichnet, dass** die Temperatur der einströmenden Frischluft (AF), die keine aktiven Stoffe enthält, und/oder die Temperatur des Gasgemischs (MG) mit einer Heiz- und Regelvorrichtung eingestellt und vorzugsweise bei ungefähr 25 °C gehalten wird.

4. Verfahren nach einem der Ansprüche 1 bis 3, **dadurch gekennzeichnet, dass** das Zusammenbringen der gasförmigen Frischluft (AF) mit dem mindestens einen flüchtigen aktiven Stoff (SA) in der Gasphase, damit die Frischluft (AF), vorzugsweise bis zur Sättigung, mit dem oder den flüchtigen aktiven Stoff (en) (SA) in der Gasphase in den Anreicherungs- oder Sättigungskammern (7) dadurch erfolgt, dass die Frischluft (AF) oder das in Schritt d) erneut eingeleitete Gasgemisch (MG) auf, unter und/oder entlang einer oder mehrerer Flächen (12) und/oder durch einen oder mehrere Volumenkörper geleitet wird, die bzw. der mit einem oder mehreren flüchtigen aktiven Stoff(en) (SA) zur Beseitigung unangenehmer Gerüche, zur Beduftung, zur Desinfektion und/oder Insektenbekämpfung versehen ist bzw. sind, die somit in Gasform diffundieren und so die Frischluft (AF) oder das erneut eingeleitete Gasgemisch (MG) anreichern oder sättigen.

5. Verfahren nach Anspruch 4, **dadurch gekennzeichnet, dass** die Fläche oder Flächen (12) und/oder der oder die Volumenkörper regelbar direkt oder indirekt mit einer regelbaren Heizvorrichtung erwärmt werden, damit so die pro Zeiteinheit und Flächeneinheit diffundierte oder entnommene Menge an flüchtigem aktivem Stoff (SA) geregelt wird.

6. Verfahren nach einem der Ansprüche 1 bis 5, **dadurch gekennzeichnet, dass** die Entnahme in Schritt c) erfolgt, wenn die Anreicherung des Gasgemischs (MG) mit dem betreffenden aktiven Stoff (SA) in der Gasphase größer gleich 50 %, vorzugsweise größer gleich 70 % und weiter bevorzugt größer gleich 95 % des theoretischen höchsten Grads der Sättigung der Luft mit dem aktiven Stoff (SA) beträgt.

7. Verfahren nach einem der Ansprüche 1 bis 6, **dadurch gekennzeichnet, dass** das Verhältnis zwischen dem einströmenden gasförmigen Frischluftstrom (AF), der keine aktiven Stoffe enthält, und dem Gasgemischstrom oder den Gasgemischströmen (MG), der bzw. die angereichert oder gesättigt ist bzw. sind und aus dem Raum (1) strömt bzw. strömen, zwischen 1,5 und 4 oder vorzugsweise zwischen 4 und 8 liegt.

8. Verfahren nach einem der Ansprüche 1 bis 7, **dadurch gekennzeichnet, dass** das Einleiten der gasförmigen Frischluft (AF), die keine aktiven Stoffe enthält, während des gesamten Anreicherungs- oder Sättigungszyklus kontinuierlich erfolgt.

9. Vorrichtung zum Durchführen des Verfahrens nach einem der vorhergehenden Ansprüche, wobei die Vorrichtung einen dichten Raum (1) umfasst, der mit mindestens einer Einströmöffnung (2) und mit mindestens einer Ausströmöffnung (3), die beide mit der Außenumgebung des Raums (1) in Verbindung stehen, und mit einer Hauptkammer (4) versehen ist, die im Bereich einer ersten Zone (4') von letzterer über die mindestens eine Öffnung (2) von mindestens einem ersten Lüfter (5) mit Frischluft (AF) von außen, die keine aktiven Stoffe (SA) enthält, versorgt wird und die im Inneren mit mindestens einem zweiten Lüfter (6) versehen ist, der die in der Hauptkammer (4) enthaltenen Gase in Richtung von mindestens einem Einlass (E) von mehreren Anreicherungs- oder Sättigungskammern (7) zur Anreicherung oder Sättigung der Gase mit flüchtigen aktiven Stoffen (SA) saugt, und ein Mittel zur Regelung des von dem mindestens einen ersten und/oder dem mindestens einen zweiten Lüfter (5, 6) erzeugten Gasstroms, **dadurch gekennzeichnet, dass** der Auslass oder die Auslässe (S) der Anreicherungs- oder Sättigungskammern (7) in eine zweite Zone (4'') der Hauptkammer (4) münden oder damit verbunden sind, die örtlich entfernt von der ersten Zone (4') und der oder den Einströmöffnung(en) (2) liegt und die im Inneren mit mindestens einem Verdichter (9) versehen ist, um mindestens einen Teil des erzeugten angereicherten oder gesättigten Gasgemischs (MG) zu entnehmen und über mindestens eine Ausströmöffnung (3) zu einer inneren Speicherkammer (8), einem Verbraucher (10) oder einem äußeren Zwischenspeicherbehälter (11) zu leiten, wobei die zweite Zone (4'') derart mit der ersten Zone (4') in Gasverbindung steht, dass eine erneute Einleitung des erzeugten angereicherten oder gesättigten Gasgemischs (MG) in die Anreicherungs- oder Sättigungskammern (7) möglich ist, gegebenenfalls mit Frischluft (AF), die keine aktiven Stoffe enthält und über die Einströmöffnung(en) (2) einströmt.

10. Vorrichtung nach Anspruch 9, **dadurch gekennzeichnet, dass** sie mindestens ein Heiz- und Temperaturregelmittel für die Frischluft (AF) von außen, die keine aktiven Stoffe (SA) enthält, und/oder für die bereits in der Hauptkammer (4) vorhandenen Gase aufweist.

11. Vorrichtung nach Anspruch 9 oder 10, **dadurch gekennzeichnet, dass** eine oder mehrere der Anreicherungs- oder Sättigungskammern (7) mindestens eine Fläche (12) und/oder mindestens einen Volumenkörper aufweist bzw. aufweisen, die bzw. der mit (einem) flüchtigen aktiven Stoff(en) (SA) zur Beseitigung unangenehmer Gerüche, zur Beduftung, zur Desinfektion und/oder Insektenbekämpfung versehen ist, damit er bzw. sie in Gasform diffundieren kann bzw. können und so die Frischluft (AF) oder das erneut eingeleitete Gasgemisch (MG) angereichert oder gesättigt wird.

12. Vorrichtung nach Anspruch 11, **dadurch gekennzeichnet, dass** eine oder mehrere der Anreicherungs- oder Sättigungskammern (7) in Form von dichten länglichen Behältern ausgeführt sind, wobei im Inneren derselben die Fläche(n) (12) und/oder der bzw. die Volumenkörper angeordnet ist bzw. sind, die mit dem bzw. den aktiven Stoff(en) (SA) versehen ist bzw. sind, verteilt über die gesamte Längsausdehnung der Behälter, vorzugsweise auf mindestens zwei ebenen und in Höhenrichtung zueinander versetzten parallelen Höhen (13, 13').

13. Vorrichtung nach Anspruch 11 oder 12, **dadurch gekennzeichnet, dass** eine oder mehrere der Anreicherungs- oder Sättigungskammern (7) Prallbleche aufweisen.

14. Vorrichtung nach einem der Ansprüche 11 bis 13, **dadurch gekennzeichnet, dass** eine oder mehrere der Anreicherungs- oder Sättigungskammern (7) einstellbare Heizmittel für die Flächen (12) oder Volumenkörper aufweisen, die mit (einem) aktiven Stoff(en) (SA) versehen sind.

15. Vorrichtung nach einem der Ansprüche 9 bis 14, **dadurch gekennzeichnet, dass** sie mehrere zweite Lüfter (6) aufweist, wobei jeder davon mit einer Anreicherungs- oder Sättigungskammer (7) verbunden ist, die ihm zugehörig ist, wobei jede Anreicherungs- oder Sättigungskammer (7) einen eigenen Auslass (S) aufweist, der in die zweite Zone (4'') mündet oder damit verbunden ist, die mit mindestens einem Verdichter (9) zum Entnehmen von mindestens einem Teil des angereicherten oder gesättigten Gasgemischs (MG) versehen ist, und ein Mittel zur Steuerung und Regelung der mehreren zweiten Lüfter (6) und der oder des Verdichter(s) (9).

16. Verwendung des Verfahrens nach einem der Ansprüche 1 bis 8, **dadurch gekennzeichnet, dass** das angereicherte oder gesättigte Gasgemisch (MG), das bei Durchführung des Verfahrens erhalten wird, in einer wirksamen Menge mit zu behandelnder verbrauchter Luft gemischt wird und so unangenehme Gerüche beseitigt werden, sie beduftet wird, gereinigt wird und/oder Insekten abgetötet werden, die sie enthält.

## Claims

1. Method for enriching, preferably saturating, ordinary atmospheric air, referred to hereinafter as fresh air (AF), with active substances (SA) present in gaseous phase, with volatile substances for deodorization, perfuming, disinfection and/or disinsection, **characterized in that** it comprises carrying out, in an airtight enclosure (1), the following operations:
a) introducing gaseous fresh air (AF) not loaded with active substances into said enclosure (1), by means of a first ventilator (5) at at least one inlet orifice (2) of said enclosure (1),
b) introducing it by at least one inlet (E) into a plurality of enrichment or saturation chambers (7), for contacting said fresh air (AF) with at least one volatile active substance (SA) present in gaseous phase in order to enrich said air, preferably to saturation, with said volatile active substance or substances (SA),
c) withdrawing, at the outlet (S) of said enrichment or saturation chambers (7), a portion of gaseous mixture (MG) enriched to a sufficient degree or saturated at the end of step b), by means of one or more compressors (9), for the purpose of storing it in an internal storage chamber (8) or of evacuating it to the outside via one or more outlet orifices (3) of said enclosure (1),
d) re-injecting the portion of gaseous mixture (MG) not withdrawn in step c) or the entirety of said enriched or saturated gaseous mixture (MG), into said enrichment or saturation chambers (7) by means of a second ventilator (6) so as to increase the degree of enrichment of said gaseous mixture (MG), to maintain it at its maximum saturation level or to lower it by mixing it, in this case, before introduction into said enrichment or saturation chambers (7), with new, unloaded fresh air (AF) introduced from the outside into said enclosure (1), where the ratio between the stream of entering unloaded gaseous fresh air (AF) and the stream or streams of enriched or saturated gaseous mixture (MG) exiting the enclosure (1) is greater than 1.5, and
e) regulating the gaseous stream generated by the first and/or second ventilator (5, 6), so as to control the amount of volatile active substance (SA) diffused or withdrawn per unit time and unit surface area.

2. Method according to Claim 1, **characterized in that** the gaseous fresh air (AF) not loaded with active substances is filtered as it is introduced into said enclosure (1).

3. Method according to Claim 1 or 2, **characterized in that** the temperature of the entering unloaded fresh air (AF) and/or the temperature of the gaseous mixture (MG) is regulated by means of a heating and regulating device, and preferably maintained at approximately 25°C.

4. Method according to any one of Claims 1 to 3,
**characterized in that** the gaseous contacting, in the enrichment or saturation chambers (7), of said gaseous fresh air (AF) with said at least one volatile active substance (SA) in gaseous phase so as to enrich said fresh air (AF), preferably to saturation, with said volatile active substance or substances (SA) in gaseous phase is accomplished by circulating said fresh air (AF) or the re-injected gaseous mixture (MG) of step d) over, under and/or along one or more surfaces (12) and/or through one or more voluminal bodies loaded with one or more volatile active substances (SA) for deodorization, perfuming, disinfection and/or disinsection which thus diffuse, in gaseous form, to enrich or saturate said fresh air (AF) or the re-injected gaseous mixture (MG).

5. Method according to Claim 4, **characterized in that** said surface or surfaces (12) and/or the voluminal body or bodies are heated directly or indirectly and regulatably by means of a regulatable heating device, so as to regulate the amount of volatile active substance (SA) diffused or withdrawn per unit time and unit surface area.

6. Method according to any one of Claims 1 to 5,
**characterized in that** the withdrawal in step c) takes place when the enrichment of the gaseous mixture (MG) with the active substance (SA) in gaseous phase in question is greater than or equal to 50%, preferably greater than or equal to 70% and more preferably greater than or equal to 95% of the maximum theoretical degree of saturation of the air with said active substance (SA).

7. Method according to any one of Claims 1 to 6,
**characterized in that** the ratio between the stream of entering unloaded gaseous fresh air (AF) and the stream or streams of enriched or saturated gaseous mixture (MG) exiting the enclosure (1) is between 1.5 and 4, or preferably between 4 and 8.

8. Method according to any one of Claims 1 to 7,
**characterized in that** the unloaded gaseous fresh air (AF) is introduced continuously throughout the enrichment or saturation cycle.

9. Device for implementing the method according to any one of the preceding claims, said device comprising an airtight enclosure (1), which is equipped with at least one inlet orifice (2) and with at least one outlet orifice (3), both of which communicate with the outside of said enclosure (1), with a main compartment (4) which is supplied, at a first zone (4') of said compartment, by said at least one orifice (2), with external fresh air (AF) not loaded with active substances (SA), by means of at least one first ventilator (5), and which is equipped, internally, with at least one second ventilator (6), which draws the gases contained in said main compartment (4) to at least one inlet (E) of a plurality of chambers for enriching or saturating (7) said gases with volatile active substances (SA), and a means for regulating the gaseous stream generated by said at least first and/or said at least second ventilator (5, 6),
**characterized in that**
the outlet or outlets (S) of said enrichment or saturation chambers (7) open out into or are connected to a second zone (4") of said main compartment (4), which is geographically distant from said first zone (4') and from the inlet orifice or orifices (2) and which is equipped, internally, with at least one compressor (9) for withdrawing at least a portion of enriched or saturated gaseous mixture (MG) formed, intended for an internal storage chamber (8), a consumer (10) or an external intermediate storage reservoir (11) via the at least one outlet orifice (3), said second zone (4'') being in gaseous communication with said first zone (4') so as to allow the enriched or saturated gaseous mixture (MG) formed to be re-injected into said enrichment or saturation chambers (7), where appropriate with the unloaded fresh air (AF) entering by the inlet orifice or orifices (2).

10. Device according to Claim 9, **characterized in that** it comprises at least one means for heating and regulating the temperature of the external fresh air (AF) not loaded with active substances (SA), and/or of the gases already present in the main compartment (4) .

11. Device according to Claim 9 or 10, **characterized in that** one or more of said enrichment or saturation chambers (7) comprise at least one surface (12) and/or at least one voluminal body that is loaded with volatile active substance(s) (SA) for deodorization, perfuming, disinfection and/or disinsection, so as to be able to diffuse, in gaseous form, for enriching or saturating said fresh air (AF) or the re-injected gaseous mixture (MG).

12. Device according to Claim 11, **characterized in that** one or more of said enrichment or saturation chambers (7) are made in the form of elongated airtight containers in whose interior are disposed the surface or surfaces (12) and/or the voluminal body or bodies loaded with active substance(s) (SA) spread over the entire longitudinal extent of said containers, preferably on at least two planar parallel levels (13, 13') which are offset from one another in terms of height.

13. Device according to Claim 11 or 12, **characterized in that** one or more of said enrichment or saturation chambers (7) exhibit chicanes.

14. Device according to any one of Claims 11 to 13,
**characterized in that** one or more of said enrichment or saturation chambers (7) exhibit regulatable means for heating the surfaces (12) or voluminal bodies loaded with active substance(s) (SA).

15. Device according to any one of Claims 9 to 14,
**characterized in that** it exhibits a plurality of second ventilators (6), each being connected to its own enrichment or saturation chamber (7), each enrichment or saturation chamber (7) exhibiting its own outlet (S) opening out into or connected to said second zone (4'') equipped with at least one compressor (9) for withdrawing at least a portion of enriched or saturated gaseous mixture (MG), and an element for controlling and managing said plurality of second ventilators (6) and said compressor or compressors (9).

16. Use of the method according to any one of Claims 1 to 8, **characterized in that** the enriched or saturated gaseous mixture (MG) obtained by the implementation of said method is mixed, in an effective amount, with the vitiated air to be treated in order to deodorize it, perfume it, sanitize it and/or free it of the insects that it contains.
